# EUROPEAN PATENT SPECIFICATION

(11) **EP 0 626 069 B1**
(45) Date of publication and mention of the grant of the patent: **19.08.1998**
(21) Application number: 93923574.3
(22) Date of filing: 28.10.1993
(51) Int. Cl.: G01N 33/543, G01N 35/00, G01N 21/00

(54) **METHOD AND APPARATUS FOR BIOAFFINITY ASSAYS**
VERFAHREN UND ANORDNUNG FÜR BIOAFFINITÄTSASSAYS
PROCEDE ET APPAREIL POUR DES ESSAIS DE BIOAFFINITE

(30) Priority: 09.11.1992 FI 925064
(43) Date of publication of application: 30.11.1994
(73) Proprietor: SOINI, Erkki, 21610 Kirjala (FI)
(72) Inventor: SOINI, Erkki, 21610 Kirjala (FI)
(74) Representative: Weisert, Annekäte, Dipl.-Ing. Dr.-Ing.
(86) International application number: FI9300442
(87) International publication number: WO9411735

(56) References cited:
- EP-A- 430 248
- FI-B- 86 229
- US-A- 5 028 545
- Dialog Information Services, File 154, Medline, Dialog Accession No. 8676896, L.J. KRICKA et al.: "Applications of a Microfabricated Device for Evaluating Sperm Function", Clin Chem Sep 1993, 39 (9) p1944-7.

## Description

This invention relates to a method for automated multiparameter bioaffinity assay using prefabricated microparticles distributed into different categories and representing different analytes,
- in which method the microparticles belonging to different categories contain different amounts of a fluorescent dye,
- in which method the microparticles of different categories are coated with biospecific reactants binding different analytes,
- in which method the microparticles of different categories are mixed in a suspension into which suspension the sample is added, into said suspension a mixture of biospecific secondary reactants labelled with a molecule generating or catalysing luminescence or a fluorescent or a phosphorescent molecule is added,
- in which method the fluorescent dye is activated and the strength of the fluorescent emission is measured separately for each particle for the identification of the microparticle category,
- and in which method the label of the biospecific secondary reactant is activated and the biospecific binding reaction is measured by means of the signal from the molecule generating or catalysing luminescence or from the fluorescent or phosphorescent molecule.

The said method is specifically used to perform and automate multiparameter assays based on the use of microparticles, especially in such as those described in US-A-5 028 545 and the like.

Immunoassay is an assay method based on bioaffinity and widely used for biologically active molecules in clinical diagnostics and in research laboratories. Another important assay method based on bioaffinity is the DNA hybridization assay. The crucial reactant in bioaffinity assays is e.g. an antibody or a DNA probe, which is most often coupled to a solid support such as the wall of a test tube. The said reactant binds the molecule to be assayed (the analyte), and the specificity of the assay is determined above all by the characteristics of said reactant. Bioaffinity assays, which will be later also called biospecific assays, often involve a second antibody or a DNA probe labelled with, e.g., a fluorescent molecule. The complex generated in the biospecific reaction, which is bound to the solid phase, and comprises both biospecific reactants, the analyte molecule and the label, is separated from free labelled reactant, or the free fraction, by, e.g., washing the free fraction away, after which the strength of the signal produced by the label is measured by a suitable microphotometric method, which in case of fluorescent labels is a fluorometer, and in case of labels emitting chemiluminescence, a luminometer.

In diagnostics, the determination of a plurality of analytes in the same sample simultaneously may be required, this being denoted by the term "multiparameter assay". The multiparameter assays described in the literature so far are limited to the simultaneous assay of two to four analytes by using respectively two to four different labels whose signals allow sufficient spectrometric resolution.

In US-A-5 028 545 a novel multiparameter assay method is described for the simultaneous determination of more than four analytes. This method makes use of microparticles (e.g., latex particles 10 to 200 micrometer) as the solid carrier, each separate particle forming a separate stoichiometric measuring basis in the respective reactant solution.

For each specified analyte microparticles are manufactured in separate batches such that different amounts of dye are added to the different batches (categories) of said particles for the purpose of identification of microparticle categories. The said dye may also be a fluorescent dye. The dye can be evenly distributed in the internal volume of the microparticle or on their surface. The reactant employed in a multiparameter assay method is a mixture of microparticles of different categories. The microparticles are, in addition, coated with the bioaffinity reactant for the specified analyte.

As described in US-A-5 028 545, the biospecific binding reaction may be measured by means of a fluorescent label. The fluorescent label may be a lanthanide chelate with fluorescent excitation states of long lifetime (longer than 1 microsecond) compared to the lifetime of background fluorescence or the lifetime of the fluorescent excitation states of conventional organic dyes used to identifying microparticles. The fluorescence produced by lanthanide chelates is measured with a time-resolved fluorometer, which allows the efficient separation of the fluorescence emitted by the lanthanide chelates from background fluorescence and from the fluorescence due to other organic dyes.

For the measurement of analyte concentrations by means of a fluorescent lanthanide chelate the time-resolved fluorometer can be used, in which the sample is excited with a short light pulse. For the measurement of the long-lived fluorescence of the lanthanide chelate and its separation from the background fluorescence of the microparticles, from scattering and from the signal emitted by the dye associated with each category, the light detector is only activated after a specified delay time following the excitation light pulse, and the short-lived fluorescence does not reach the light detector. The identification of the microparticle, on the other hand, takes place immediately during the excitation light pulse, or the system incorporates a separate photometric detector measuring light absorption or short-lived fluorescence.

The advantage of time-resolved fluorometry resides in the fact that the signal from the dye used for identification in no way interferes with the measurement of the signal from the lanthanide chelate, because the measurements are taken at different time points. This is why the system allows for the measurement of a plurality of analytes using microparticles in the same suspension.

An alternative label for detection of the bioaffinity reaction is the use of phosphorescent molecules. Certain metalloporphyrins, including platinum-coproporphyrin and palladium-coproporphyrin have been recently found to emit phosphorescence in water solution and introduced for use for labelling of biomolecules (A. P. Savitsky & al, Dokl. Acad. Nauk. USSR, 304, 1005, 1989). The time resolved measuring principle and the device needed for detection of the phosphorescence is essentially similar with the device normally used for detection of long decay time fluorescence of lanthanide chelates.

Alternatively, a biospecific binding reaction may be measured also by means of a luminescent label, the said lanthanide chelate being replaced by a luminescent label. The luminescent label may be in the form of a plurality of already known chemiluminescent or bioluminescent molecules. Examples of these are, among others, luciferase enzyme, derivatives of luminol, or the like. In this case microparticles are identified by a separate detector measuring light absorption or fluorescence, before the activation of the luminescent reaction. The dye used for identification does not interfere in any way with luminescence measurement, because the excitation and emission mechanisms are totally different and occur at different time points in the two cases.

Because each microparticle reacts on its own stoichiometrically, it is important that the particles be of exactly the same size. It is possible today to manufacture microparticles of precisely the same size for this purpose. If, however, the size distribution is too wide, an imaging light detector (e.g., a CCD element), may be used to identify the microparticles, by separate measurement of the diameter of each microparticle. The result is then utilized to perform the appropriate correction of the measurement data.

The aforementioned US-A-5 028 545 does not describe the actual manner to carry out and automate this multiparameter assay based on bioaffinity. In general, fluid handling in the clinical and other analyzers ("auto-analysers") in use today is based on the use of test-tubes or microtiter plates, or fluids are handled in tubings controlled by peristaltic and piston pumps and various mechanical valves, in which tubings different samples are separated from each other by means of air bubbles, during the dispensing and transport of fluids. These apparatuses are characterized by lack of provision for handling and transporting fluid volumes of less than 10 microliter or transporting microparticles one at a time at a specified speed. If in these apparatuses the internal diameter of the tubing is reduced to be almost equal to the external diameter of the microparticles, these microparticles may compromise the reliable operation of the mechanical valves. The fluid volumes handled in these apparatuses are generally at least tens of microliters, and cross-contamination is of the order of 0.1 to 1%. The concentration dynamics required in assays based on bioaffinity is at least 1000 times or, most often, 10000 times, and the use of conventional fluid handling elements and tubings in the field of application of this invention is made unacceptable by such cross-contamination levels.

Part of the fluid handling in conventional analyzers is also done in an open system. An open system in this connection denotes a system in which the samples or the reactants are, at some point of the assay, in open containers from which they may be dispensed or diluted or in which they may be incubated or measured. Examples of open analysis systems in general use are those in which samples are handled in test-tubes or microtiter plates. A closed analysis system in this connection denotes a system in which the samples and reactants are not contained in open vessels during the assay, nor are they in contact with air external to the system or with a gaseous phase in equilibrium with air. The problem with an open system is exposure of the samples and reactants to the chemical environmental factors and to evaporation as well as exposure of the personnel to possible pathogens in the samples. In addition, the surface tension of the fluids restricts the possibility to dispense microvolumes of 1 microliter or less, and in practice the sample volumes in all open systems during the process are more that 10 microliter or even more than 100 microliter.

The multiparameter assay method based on bioaffinity and the use of microparticles, described in US-A-5 028 545, cannot be automated by applying the aforementioned conventional principles, because open fluid handling systems do not allow for precise transfer or dispensing of single or a small number of microparticles contained in volumes smaller than 1 microliter. The object of this invention is to describe the manner in which to construe an automated multiparameter bioaffinity microassay system comprising microparticles as a solid reaction support and a system of microchannels for fluid handling. A system of microchannels in this connection denotes channels wider by, e.g., twice the external dimensions of the microparticles used for this purpose, the said channels being in the form of either thin tubes or thin channels manufactured inside a solid support by etching or engraving or by some other suitable method, the said channels connecting separate fluid handling operations, and along which channels suspensions of microparticles are transferred from one point to another. The microchannels have a geometric design such as allows the transfer and handling of microparticles in the form of a fluid suspension while avoiding obstruction of the system of channels by the particles or their remaining in pockets of the system of channels. The internal dimensions of the system of microchannels are selected such as to allow movement of microparticles in said system of microchannels, centered by laminar flow, in the center of the microchannel, singly in a row. Those parts of the system of microchannels which are intended for storing samples or for incubation, e.g., the sample register, may, however, be of wider internal dimensions. If rather large microparticles, e.g., larger than 100 micrometer, are used, a suitable internal diameter is 250 micrometer for a microchannel intended for the transfer of a suspension of microparticles. If smaller microparticles, e.g., of the size of 10 micrometer, are used, a suitable internal diameter is e.g. 50 micrometer for a microchannel intended for the transfer of a suspension of microparticles.

The purpose of the use of a system of microchannels is to use microvolumes and at the same time make savings in the expenditure for reagents. Cost savings are realized by reducing the amounts of specific reactants to a fraction, e.g., a hundredth of the volumes used in modern open systems. In a closed analysis system designed according to this invention, based on a system of microchannels, samples of a volume less than 1 microliter or even less than 100 nanoliter may be handled quantitatively with good precision. In addition, this invention allows a plurality of simultaneous different bioaffinity determinations, that is, a specified diagnostic panel, or the combination of all the analytes relevant to specified diagnostic symptoms, for instance, to be carried out in the said volume. In practice a capacity of ten analytes is probably sufficient, although the said system probably allows an even greater number of analytes to be measured simultaneously. The adoption of the said microassay system possibly allows routine diagnostics to be simplified and the extent of manual handling and the amounts of reactants and packaging material to be reduced. In reagent production the said system will also allow great economies. The production of diagnostic test kits for open systems involves bulky fluids, coated test-tubes and their packaging and also requires a high standard of hygiene. The production system for reactants for a closed microassay system such as described here may be construed as a closed process.

The object of this invention is to describe the manner in which an assay method of the aforesaid characteristics can be completely automated by applying the concept of a system of microchannels.

This object is achieved by a method of the type mentioned in the beginning, which is **characterized** by the use of a system of microchannels in the assay for the identification of microparticle categories and the measurement of biospecific binding reactions and possibly also for dispensing, mixing, incubation or the separation of the free fraction.

Microparticles have been observed to move in the microchannels, centered by laminar flow, onto the center axis of the channel, one after the other, in a controlled manner. All stages of handling of microparticles are carried out in a system of microchannels according to this invention, including at least the identification of categories and measurement, and according to the capacity of the apparatus, possibly also dispensing, mixing, incubation, and the separation of the free fraction. A sample register is formed by the microchannels and valves to allow simultaneous incubation of a plurality of samples.

Mixing of samples and reactants in connection with dispensing and during incubation is effected by turbulent flow generated by hydraulic pressure pulses in the microchannel, which contains, in addition to the aforementioned fluids, a gas phase which provides an elastic cushion to receive the pressure pulses. Mixing is effected in such a manner that the fluids are not in contact with moving wear-sensitive parts. The free and the bound labelled reactant are separated by exchanging the liquid phase in the microchannel containing the microparticles.

One system of microchannels satisfying the aforesaid requirements is the system of "microfluidistics" whose operational principles and components have been described in, among others, the following Finnish patents: Nos. 56591, 57849, 57850, 64012, 67490, 70331, 71102, 72660, and 86229. After the publication of the aforementioned patents more sample handling methods, components and their production methods based on microfluidistics have been developed, such as a dispensing needle for the sterile recovery of the sample from the primary sample tube, and a method for the manufacture of the said needle, a method for the mixing of a fluid volume in a vessel during dispensing by means of the dispensing needle, and a nonlinear dispenser based on a metal bellows, the accuracy of the dispenser being constant at all volumes.

The microfluidistics system described in the aforementioned patents allows precise handling and transport of small reactant volumes (less than 1 microliter) and small numbers of microparticles or even of single particles, in a closed system of microchannels. The said system is a hermetically sealed, sterile if need be, quantitative system for fluid handling, composed of thin-walled microchannels of inert metal. Fluid handling is directed through electrically controlled ice valves, in which valves the fluid (sample, reactant or auxiliary fluid) is frozen or defrosted to close or to open the parts functioning as gates for the microchannels. The volume of the valves is only, e.g., 50 nanoliters, which means that they have a reduced heat capacity and consequently their operation is fast. An important advantage of the said ice valves in a system designed according to this invention is provided by the fact that the microparticles do not interfere mechanically with the functioning of the ice valves, even though their external dimensions were almost equal to the internal dimensions of the microchannel.

An alternative technology of manufacturing microfluidistics is the use of crystalline silicon as solid substrate. The microchannels and other necessary structures can be etched in this silicon using a planar photolitographic micromachining techniques developed by microelectronics industry (M. Salonen and T. Ropponen, Mikromekaniikka, Helsinki University of Technology, Publication no. C234, 1991). Standars silicon wafers with a suitable crystalline orientation and diameter e.g. 100 mm or larger can be used. The connections can be etched into the silicon wafer on either side and the liquid is supplied or removed from the microchannel system through vertical bores.

The upper face of the silicon wafer consists of thin quarz glass which is hermetically attached to silicon using standard heating technique. The quarz cover permits direct flow observation and detection of photon emission from the luminescent, fluorescent or phosphorescent labels of the reactants on the microparticles. Silicon wafers used by semiconductor industry is chemically extremely pure and according to our test results does not contain any impurities, e.g. heavy metals which would interfere with the fluorescence detection of the lanthanide chelates. Certain types of quarz also fulfill the purity requirements.

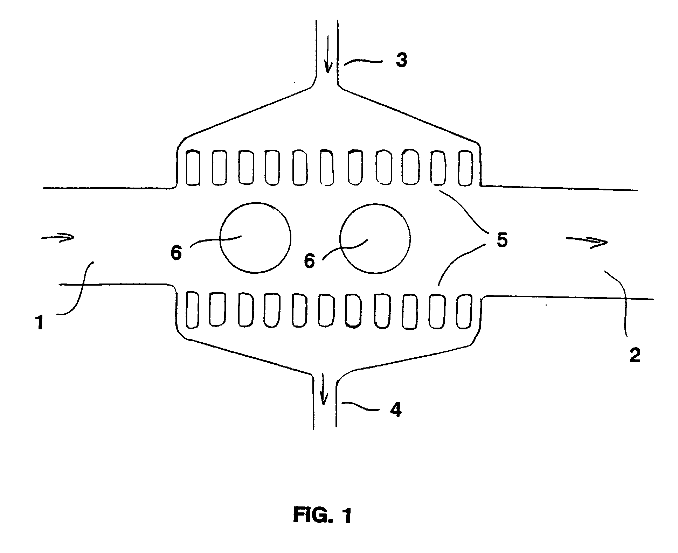

Microchannels for liquid handling can be etched in silicon with precision of 0.1 micrometers (J. Phaler & al., Liquid transport in micron and submicron channels, Sensors and Actuators, A21 A23 (1990) 431-434). This precision permits constructions of reaction chambers where the microparticles are kept inside a geometrically precisely defined part of a microchannel whose wall is a filter. The principal construction of a such reaction chamber is shown in Figure 1. This construction allows washing of the free fraction of the labelled reactants without moving the microparticles. The microparticles are fed in through the input 1. and fed out through the output 2. The washing is performed simply by a flow of washing solution through the filter from the input 3 and the output 4. The filter 5 prevents the microparticles 6 to move in the direction of the wash flow. The filter consists of parts of the silicon having any optimal form and dimensions. It is obvious that the spacing between the parts shall be smaller than the diameter of the microparticle.

Most of the microfluidistics functions described in the patents referred above, can be performed also by the constructions in silicon, including for example ice valves. Silicon technology allows to integrate silicon with metal constructions and metals with high thermal conductivity can be used as "cold fingers" for operating the ice valves. High thermal insulation between the cold fingers and other construction can be achieved with built-in vacuum cavities.

Silicon technology allows to integrate micropump membranes, heating elements, temperature exchangers, temperature sensors, pressure sensors, optical sensors, other optical elements and electronics in the same silicon wafer with the microfluidistics.

The principal characteristics of the microfluidistics described above are the following:
- the fluid handling spaces are of such geometrical design that the microparticles can be moved at all times by a hydraulic mechanism, and the system does not contain pockets the size of or larger than the particle dimensions,
- the construction of the system is hermetic and sterile, the inputs and the outputs being subjected to biosafety control,
- the volume of liquid waste, sterilizable internally, is less than 10 - 100 microliter per analysis,
- the fluid is not in contact with any separate moving, wear-sensitive components,
- the dispensing system of the fluids is completely hydraulic (no liquid-gas interphase),
- the fluids are dispensed within a volume range of 1 nanoliter - 100 microliter),
- the accuracy of the handling of fluids is 0.1 % in the microlitre range and 1 - 5 % in the nanolitre range,
- the decontamination capacity of the liquid handling system (cross-contamination) is 0.0001 %,
- hydraulic mixing is provided for the reactants during dispensing and incubation, and the fluids to be mixed are not in contact with moving, wear-sensitive parts.

The complete automation described here comprises an apparatus into which fluid samples, e.g., blood serum, are fed through a thin tube. The necessary reactants, e.g., the suspension of microparticles and the necessary buffers and wash liquids are contained in purposeful and sterile (if needed) containers hermetically connected to the apparatus.

Samples and reactants are handled in the said system in a closed space and in microvolumes. The essential reactants of the said system are the following:
1. The microparticle suspension is a mixture of microparticles coated with different bioaffinity reactants, the diameter of the said particles being 1 - 200 micrometer. An example of commercial microparticles is the selection of Duke Scientific Inc. (USA). Magnetic microparticles can also be used.
2. A buffer is a fluid used to optimize the chemical environment during incubation. A buffer may also be used to wash the system.
3. A measuring fluid is a fluid used to optimize the chemical environment during the measurement of the fluorescence of the lanthanide chelate or during the measurement of phosphorescence or luminescence. A measuring fluid may incorporate an enhancer of the fluorescence of the lanthanide chelate (e.g. cofluorescence) and a reagent to eliminate agglutination of the microparticles.

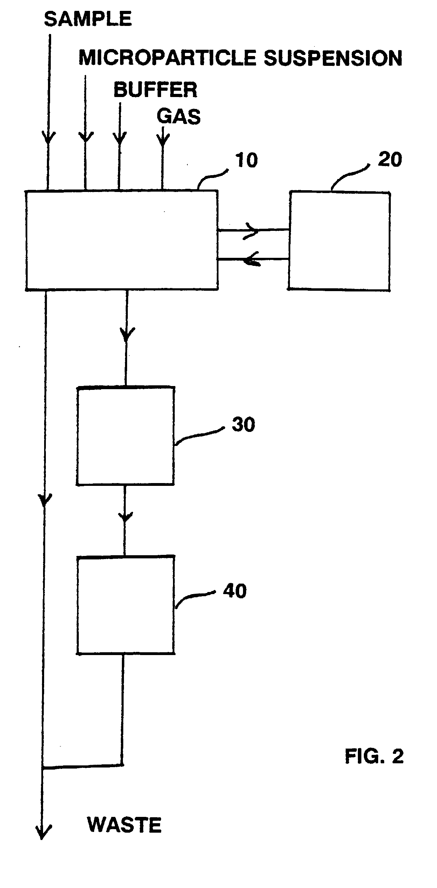

A functional block diagram of the system is shown in Figure 2, comprising the following parts:
1. A multiplace incubator 10, into which the sample, microparticle suspension and buffer are fed. The multiplace incubator incorporates microchannels, ice valves and temperature regulators, and allows random input of samples and simultaneous incubation. In the incubator the sample is mixed with the microparticle suspension. The incubation time of each sample is precisely controlled, and the bioaffinity reaction is not necessarily brought to equilibrium. The number of microparticles being in the incubator at any given time does not have a significant effect on reaction kinetics and precision but the system allows a precise count of the microparticles to be taken, should that prove useful for the sake of precision. Different samples may be incubated for different lengths of time.
   Bound reactant is separated from free reactant by, e.g., the well-known by-pass washing-filtering method. An alternative method is to fix the microparticles in the incubator by means of magnetic field and wash the free reactant away, at the same time replacing the free reactant with the measuring fluid. The closed flow-through centrifuge described in FI patent No. 72660 or the filter method described in FI patent No. 67490 may also be used for said separation.
2. A pump 20 forthe transfer of fluids in the microchannels and of the microparticle suspension into the detector. Pumping continues until the desired number of microparticles corresponding to each category have been measured. The micropump ensures the transport of the suspension into the detectors at precisely controlled speed, and the microparticle may be moving or stationary while measurement is taking place. The microparticle suspension is at this stage so dilute that the average inter-particle distance in the flow capillary is sufficient to allow the optical resolution of the signals they emit. The micropump moves the microparticle suspension in the microphotometric measuring channel at a predetermined speed along the center axis of the channel, the microparticles being centered by laminar flow, and the location of the particle may be determined in the direction of the flow, if necessary, with the precision of micrometers, at the focal point of the photometric detector. The liquid flow centers the particle in the transverse direction of the flow with the same precision. If the specific mass of the particle differs from that of the measuring fluid, the particle will, after being localized, that is, after liquid flow has stopped, either settle or rise against the wall of the horizontal measuring channel in a fraction of a second. At this point a photometric, fluorometric or luminometric measurement of the particle may be taken with the accuracy and sensitivity required for the particular application.
   The micropump described above may be, e.g., a bellow pump controlled by a computer-controlled stepping motor and an eccentric drive, the volume of fluid dispensed by the pump having a nonlinear relation to the rotation angle of the stepping motor, which allows transport of widely differring fluid volumes at a predetermined speed and with constant precision.
3. The element 30 used for identifying the microparticles is a photometric or fluorometric device incorporating a light source and a photodetector for the measurement of the dye contained in the microparticle and possibly for the measurement of the diameter of the microparticle. The category of each microparticle is identified by the system according to the strength of the signal from the detector. The information obtained from the identification process and from the measurement is used as calculation parameters in the standardization program for the particular analyte in a computer which calculates the concentration of the analyte in the sample based on the signal strength obtained from the detector (40) for the label. The identifying element can also detect agglutinated microparticles, which are not measured but are diverted away.
4. The detector 40 for a label is either a time-resolved fluorometer or phosphorometer or a luminometer. The measuring cell may be a commercial flow-through cell measuring, e.g., 0.25 by 0.25 mm in cross section and with an effective volume of less than 20 nanoliter, in which volume the signal of just one microparticle is measured at a time. Alternatively the measuring chamber is integrated in same silicon substrate with other functional parts.
The system further incorporates for the control of the functional elements for fluid handling and for the control of the electrical functions, other required functions such as conventional pumps, valves, fluid containers and temperature regulators as well as the required number of electronic control elements and a computer for system control and for the standardization and computation of results.

The described embodiments are illustrative and should not be construed as restrictive.

## Claims

1. A method for automated multiparameter bioaffinity assay using prefabricated microparticles (6) distributed into different categories and representing different analytes,
- in which method the microparticles (6) belonqinq to different categories contain different amounts of a fluorescent dye,
- in which method the microparticles (6) of different categories are coated with biospecific reactants binding different analytes,
- in which method the microparticles (6) of different categories are mixed in a suspension into which suspension the sample is added, into said suspension a mixture of biospecific secondary reactants labelled with a molecule generating or catalysing luminescence or a fluorescent or a phosphorescent molecule is added,
- in which method the fluorescent dye is activated and the strength of the fluorescent emission is measured separately for each particle (6) for the identification of the microparticle category,
- and in which method the label of the biospecific secondary reactant is activated and the biospecific binding reaction is measured by means of the signal from the molecule generating or catalysing luminescence or from the fluorescent or phosphorescent molecule, **characterized** by the use of a system of microchannels in the assay for the identification of microparticle categories and the measurement of biospecific binding reactions and possibly also for dispensing, mixing, incubation or the separation of the free fraction.

2. The method according to claim 1, **characterized** by fluid transport in thin-walled microchannels manufactured of inert metal by electroformation or by fluid transport in microchannels made into a substrate manufactured with lithography and etching.

3. The method according to claim 1, **characterized** by the use of a closed design of the fluid handling system formed by microchannels.

4. The method according to claim 1, **characterized** by the use of ice valves for the control of the operation of the system of microchannels and/or dispensing of fluids.

5. The method according to claim 1, **characterized** by the use of hydraulic dispensing of fluids so that essentially no liquid-gas interface exists in the microchannels during dispensing.

6. The method according to claim 1, **characterized** by a control of the flow of the microparticles in the system of microchannels, at varying speed, determined by a micropump (20) precisely controlled by a computer.

7. The method according to claim 6, **characterized** by identification and measurement of the movement of the microparticles (6) by means of microphotometric detectors and feeding the resulting data into the computer controlling the pump (20).

8. The method according to claim 6, **characterized** by the use of a micropump (20) being a nonlinear bellows pump with constant precision over a wide range of volumes.

9. The method according to any one of the claims 1 - 8, **characterized** by the use of a sample register formed by microchannels and valves, for simultaneous incubation of the samples.

10. The method according to any one of the claims 1 - 9, **characterized** by separation of free and bound labelled reactants by extensive dilution or by exchanging the fluid phase containing the microparticles (6).

11. The method according to claim 10, **characterized** by optimization of the new fluid phase resulting from exchange or dilution, to provide maximal assay sensitivity of the label.

12. The method according to claim 10, **characterized** by separation of the free reactant from the microparticles (6) by dilution or by-pass flow filtering in a microchannel containing a plane or a device to detain the microparticles (6).

13. The method according to claim 12, **characterized** by the use of a device being a closed flow-through centrifuge.

14. The method according to any one of the claims 1 - 13, **characterized** by mixing of samples and reactants during dispensing and incubation by means of turbulent flow generated by hydraulic pressure pulses in a microchannel containing, in addition to the aforementioned fluids, a gas phase for the elastic reception of the pressure pulses.

15. The method according to any one of the claims 1 - 14, **characterized** by decontamination of the system of microchannels after preceding sample by pumping a mixture of fluid and gas through the system of microchannels.

## Patentansprüche

1. Verfahren für ein automatisiertes Multiparameter-Bioaffinitäts-Assay unter Verwendung von vorgefertigten Mikropartikeln (6), die in unterschiedliche Kategorien aufgeteilt sind und unterschiedliche Analyte repräsentieren,
- in welchem Verfahren die Mikropartikel (6), die zu unterschiedlichen Kategorien gehören, unterschiedliche Mengen eines fluoreszenten Farbstoffs enthalten,
- in welchem Verfahren die Mikropartikel (6) von unterschiedlichen Kategorien mit biospezifischen Reaktanten beschichtet sind, die unterschiedliche Analyte binden,
- in welchem Verfahren die Mikropartikel (6) von unterschiedlichen Kategorien zu einer Suspension gemischt werden, zu welcher Suspension die Probe hinzugefügt wird, wobei zu der Suspension eine Mischung von biospezifischen sekundären Reaktanten hinzugefügt wird, die mit einem Molekül, das Lumineszenz erzeugt oder katalysiert oder einem fluoreszenten oder einem phosphoreszenten Molekül markiert sind,
- in welchem Verfahren der fluoreszente Farbstoff aktiviert und die Stärke der fluoreszenten Emission separat für jedes Partikel (6) zur Identifikation der Mikropartikelkategorie gemessen wird,
- und in welchem Verfahren die Markierung des biospezifischen sekundären Reaktanten aktiviert und die biospezifische Bindungsreaktion mittels des Signals von dem Molekül, das Lumineszenz erzeugt oder katalysiert, oder von dem fluoreszenten oder phosphoreszenten Molekül gemessen wird, **gekennzeichnet** durch die Verwendung eines Systems von Mikrokanälen in dem Assay für die Identifikation der Mikropartikelkategorien und die Messung der biospezifischen Bindungsreaktionen und vielleicht auch für das Verteilen bzw. Ausgeben, das Mischen, die Inkubation oder die Trennung der freien Fraktion.

2. Verfahren gemäß Anspruch 1, **gekennzeichnet** durch Fluidtransport in dünnwandigen Mikrokanälen, die aus inertem Metall durch Elektro- bzw. Galvanoformung hergestellt sind, oder durch Fluidtransport in Mikrokanälen, die zu einem Substrat gemacht sind, hergestellt mit Lithographie und Ätzen.

3. Verfahren gemäß Anspruch 1, **gekennzeichnet** durch die Verwendung einer geschlossenen Ausführung des Fluidhandhabungssystems, das von Mikrokanälen gebildet ist.

4. Verfahren gemäß Anspruch 1, **gekennzeichnet** durch die Verwendung von Eisventilen für die Steuerung bzw. Regelung des Betriebs des Systems der Mikrokanäle und/oder das Verteilen bzw. Ausgeben von Fluiden.

5. Verfahren gemäß Anspruch 1, **gekennzeichnet** durch die Verwendung des hydraulischen Verteilens bzw. Ausgebens von Fluiden so, daß im wesentlichen keine Flüssigkeits-Gas-Grenzfläche während des Verteilens bzw. Ausgebens in den Mikrokanälen vorhanden ist.

6. Verfahren gemäß Anspruch 1, **gekennzeichnet** durch die Steuerung bzw. Regelung der Strömung der Mikropartikel in dem System von Mikrokanälen bei bzw. mit variierender Geschwindigkeit, bestimmt durch eine Mikropumpe (20), die präzise mittels eines Computers gesteuert bzw. geregelt wird.

7. Verfahren gemäß Anspruch 6, **gekennzeichnet** durch die Identifikation und Messung der Bewegung der Mikropartikel (6) mittels mikrophotometrischer Detektoren und Zuführen der resultierenden Daten in den Computer, welcher die Pumpe (20) steuert bzw. regelt.

8. Verfahren gemäß Anspruch 6, **gekennzeichnet** durch die Verwendung einer Mikropumpe (20), die eine nichtlineare Balgpumpe ist, mit konstanter Präzision über einen weiten Bereich von Volumina.

9. Verfahren gemäß irgendeinem der Ansprüche 1 bis 8, **gekennzeichnet** durch die Verwendung eines von Mikrokanälen und Ventilen gebildeten Probenregisters für die gleichzeitige Inkubation der Proben.

10. Verfahren gemäß irgendeinem der Ansprüche 1 bis 9, **gekennzeichnet** durch die Trennung von freien und gebundenen markierten Reaktanten mittels extensiver Verdünnung oder durch Austauschen der Fluidphase, welche die Mikropartikel (6) enthält.

11. Verfahren gemäß Anspruch 10, **gekennzeichnet** durch die Optimierung der neuen Fluidphase, die aus dem Austausch oder der Verdünnung resultiert, um eine maximale Assay-Empfindlichkeit der Markierung vorzusehen.

12. Verfahren gemäß Anspruch 10, **gekennzeichnet** durch die Trennung des freien Reaktanten von den Mikropartikeln (6) mittels Verdünnung oder Umleitungs- bzw. Nebenschlußströmungsfilterung in einen Mikrokanal, der eine Ebene oder eine Einrichtung zum Zurückhalten der Mikropartikel (6) enthält.

13. Verfahren gemäß Anspruch 12, **gekennzeichnet** durch die Verwendung einer Einrichtung, die eine geschlossene Durchflußzentrifuge ist.

14. Verfahren gemäß irgendeinem der Ansprüche 1 bis 13, **gekennzeichnet** durch Mischen von Proben und Reaktanten während des Verteilens bzw. Ausgebens und der Inkubation mittels turbulenter Strömung, die durch Hydraulikdruckimpulse in einem Mikrokanal erzeugt wird, der zusätzlich zu den vorerwähnten Fluiden eine Gasphase für die elastische Aufnahme der Druckimpulse enthält.

15. Verfahren gemäß irgendeinem der Ansprüche 1 bis 14, **gekennzeichnet** durch die Dekontamination des Systems von Mikrokanälen nach einer vorhergehenden Probe durch Pumpen einer Mischung von Fluid und Gas durch das System der Mikrokanäle.

## Revendications

1. Procédé pour un essai de bioaffinité automatisé et à paramètres multiples, utilisant des microparticules préfabriquées (6) réparties en différentes catégories et représentant des analytes différents,
- procédé dans lequel les microparticules (6) appartenant aux différentes catégories contiennent différentes quantités d'un colorant fluorescent,
- procédé dans lequel les microparticules (6) appartenant aux différentes catégories sont recouvertes de réactifs biospécifiques se liant aux différents analytes,
- procédé dans lequel les microparticules (6) des différentes catégories sont mélangées dans une suspension dans laquelle l'échantillon est ajouté, suspension dans laquelle un mélange de réactifs biospécifiques secondaires marqués avec une molécule générant ou catalysant la luminescence ou avec une molécule fluorescente ou phosphorescente est ajouté,
- procédé dans lequel le colorant fluorescent est activé et l'intensité de l'émission fluorescente est mesurée séparément pour chaque particule (6) en vue de l'identification de la catégorie de microparticule,
- et procédé dans lequel le marqueur du réactif biospécifique secondaire est activé et la réaction de liaison biospécifique est mesurée à l'aide du signal venant de la molécule générant ou catalysant la luminescence ou provenant de la molécule fluorescente ou phosphorescente , caractérisé par l'utilisation d'un système de microcanaux dans l'essai destiné à l'identification des catégories de microparticule et la mesure des réactions de liaison biospécifique et , éventuellement, également à la distribution, au mélange , à l'incubation ou à la séparation de la fraction libre.

2. Procédé selon la revendication 1, caractérisé par un transfert de fluide dans des microcanaux à paroi mince constitués d'un métal inerte par électroformation ou par un transfert de fluide dans des microcanaux réalisés dans un substrat fabriqués par lithographie et gravure.

3. Procédé selon la revendication 1, caractérisé par l'utilisation d'un concept en milieu fermé du système de traitement de fluide constitué de microcanaux.

4. Procédé selon la revendication 1 , caractérisé par l'utilisation de soupapes à glace destinées au contrôle du fonctionnement du système de microcanaux et/ou de distribution des fluides.

5. Procédé selon la revendication 1, caractérisé par l'utilisation d'une distribution hydraulique des fluides de sorte qu'aucune interface liquide-gaz n'existe essentiellement dans les microcanaux pendant la distribution.

6. Procédé selon la revendication 1, caractérisé par une commande de l'écoulement des microparticules dans le système de microcanaux, à vitesse variable, déterminé par une micropompe (20) commandée de façon précise par un ordinateur .

7. Procédé selon la revendication 6, caractérisé par l'identification et la mesure du déplacement des microparticules (6) au moyen de détecteurs microphotométriques et fournissant les données résultantes à l'ordinateur commandant la pompe (20).

8. Procédé selon la revendication 6, caractérisé par l'utilisation d'une micropompe (20) qui est une pompe à soufflet non linéaire ayant une précision constante sur une gamme importante de volumes .

9. Procédé selon l'une quelconque des revendications 1 à 8, caractérisé par l'utilisation d'un registre d'échantillons formé par des microcanaux et des soupapes , pour une incubation simultanée des échantillons .

10. Procédé selon l'une quelconque des revendications 1 à 9, caractérisé par la séparation des réactifs marqués libres et liés à l'aide d'une dilution étendue ou d'un échange de la phase fluide contenant les microparticules (6).

11. Procédé selon la revendication 10, caractérisé par l'optimisation de la nouvelle phase fluide provenant de l'échange ou de la dilution afin de fournir une sensibilité maximale d'essai du marqueur.

12. Procédé selon la revendication 10, caractérisé par la séparation du réactif libre des microparticules (6) par dilution ou filtrage de l'écoulement en dérivation dans un microcanal contenant un plan ou un dispositif pour retenir les microparticules (6).

13. Procédé selon la revendication 12 , caractérisé par l'utilisation d'un dispositif qui est un dispositif de centrifugation hermétique à écoulement transversal.

14. Procédé selon l'une quelconque des revendications 1 à 13 caractérisé par le mélange des échantillons et des réactifs pendant la distribution et l'incubation au moyen d'un écoulement turbulent généré par des impulsions de pression hydraulique dans un microcanal contenant, en plus des fluides mentionnés ci-dessus, une phase gazeuse destinée à la réception élastique des impulsions de pression.

15. Procédé selon l'une quelconque des revendications 1 à 14 , caractérisé par une décontamination du système de microcanaux après l'échantillon précédent par pompage d'un mélange de fluide et de gaz à travers le système de microcanaux.
